# EUROPEAN PATENT SPECIFICATION

(11) **EP 3 589 190 B1**
(45) Date of publication and mention of the grant of the patent: **28.12.2022**
(21) Application number: 18716649.1
(22) Date of filing: 01.03.2018
(51) Int. Cl.: A61B 5/00, A61B 5/01

(54) **MULTIMODAL MEDICAL IMAGING AND ANALYZING SYSTEM, METHOD AND SERVER**
MULTIMODALES SYSTEM ZUR MEDIZINISCHEN BILDGEBUNG UND ANALYSE, VERFAHREN UND SERVER
SYSTÈME, PROCÉDÉ ET SERVEUR D'IMAGERIE MÉDICALE MULTIMODALE ET D'ANALYSE

(30) Priority: 01.03.2017 FI 20175190
(43) Date of publication of application: 08.01.2020
(73) Proprietor: TherMidas Oy, 33210 Tampere (FI)
(72) Inventor: IHME, Jouni, 90940 Jääli (FI); ALASAARELA, Esko, 90540 Oulu (FI); ILO, Arjaleena, 33210 Tampere (FI); LUTTINEN, Rami, 71910 Alapitkä (FI)
(74) Representative: Berggren Oy
(86) International application number: PCT/FI2018/050152
(87) International publication number: WO 2018/158504

(56) References cited:
- CN-A- 106 327 579
- US-A1- 2001 046 316
- US-A1- 2009 028 408
- US-A1- 2009 226 068
- US-A1- 2012 093 382
- US-A1- 2014 243 683
- US-A1- 2015 018 691
- US-A1- 2016 364 857
- AHMAD GHAFARPOUR ET AL: "A review of the dedicated studies to breast cancer diagnosis by thermalimaging in the fields of medical and artificial intelligence sciences", BIOMEDICAL RESEARCH, vol. 27, no. 2, 1 January 2016 (2016-01-01), pages 543-552, XP055450277, India ISSN: 0970-938X

## Description

### Background of the invention

Diabetes causes many health concerns and physical implications with patients diagnosed with it, and many patients remain undiagnosed as well. Obesity is one characteristic for patients, and also blood circulation disorders are common. In limb areas, such as legs, the blood circulation may be weakened and as a result of that, touch sensitivity may be reduced, and there is a risk to obtain diabetic lesions on the skin. At a worse case, the blood circulation may diminish in the legs to an extent, where there is a risk to leg damage, and even more severely, a risk of need for an amputation remains there if the blood circulation is functioning very badly for a long time.

The temperature of the surface of the leg skin, and its inner tissue is of outermost importance in this regard. Traditionally, the temperature values and possible differences between the two legs have been examined through palpation, where the nurse or doctor performs a manual and subjective examination by holding and sensing the legs by the nurse's or doctor's hands. Such a manual method is quite inaccurate, and the results also depend heavily on the temperature of the nurse's or doctor's own hands.

Another traditional method is to use a thermometer for performing a skin surface temperature measurement on the leg tissue, and by choosing the measurement spots on various surface locations in order to obtain a group of results, which results could afterwards be averaged. However, this method has the same problems as the fever measurement in the armpit, and it is not an accurate measurement method.

There are also numerous other diseases, which affect the skin surface and tissue temperatures especially in the limbs of the human patient. These diseases comprise neurological diseases or disorders associated with Charcot, the French neurologist, such as Charcot-Marie-Tooth disease. Also joint inflammation, erysipelas, Raynaud syndrome, peripheral arterial block etc. causes local temperature changes.

Among patent publications, US 8,923,954 ("Herman") discloses a thermal full-body measurement system for detecting skin lesions and even possible skin cancers along the whole human skin area. The presented tracking system scans the whole body starting from the top and going downwards. It comprises thermal infrared cameras, and also regular white-light cameras, in separate linear arrangements of the scanning frame. Thermal and regular images are captured and interesting areas may be picked and forwarded into a further analysis. This publication applies thermodynamic models, which correspond to various types of notable skin changes or skin cancers.

US 8,971,984 discloses an IR optical scanner, which is used to examine feet of a diabetic patient, for instance. The IR scan reveals the outer edges of the leg, and e.g. the height and curvature of the plantar arch. This method also performs a stress test for the leg to be examined.

US 8,918,162 captures thermal IR images from the face of the person to be examined. They can be taken in a 3-dimensional mode obtaining images from various directions of the human face. Surface temperatures in different places of the human face can be detected and mapped. Changes in the temporally placed sequence of subsequent images are searched, and the magnitude of the changes can be examined as well, in order to determine a psychological state of the examined person.

EP 1250088 discloses a joint and tissue examination method, which applies several various sensors. There is a glossmeter which is a light-sensitive sensor observing the color of the skin. Furthermore, there is a handheld pressure probe (a dolorimeter), which detects the pain threshold of the examined area. Furthermore, there is performed a temperature measurement through IR sensors. The result is a calculated estimate of an inflammation value, which can be compared to a set threshold value. Diabetes nerve damage has been generally mentioned as an example of the examined situation within the patient.

Fluke Corporation has presented a concept called "Fluke IR-Fusion Technology". Their apparatus is a handheld measurement tool for checking various structures and locations within buildings in order to find thermal leak spots in them. The tool has a grip and a display where the optical image is shown. In the center of the optical image, a part of the image space is topped by an IR thermal image showing the received thermal energies. The IR image is a rectangle fully inside the edges of the optical image. Fluke's patent US 7,538,326 ("Johnson") discloses such a visible light and IR combined image camera with a laser pointer.

Furthermore, US 9,413,988 ("Heinke") discloses a thermal imaging camera with graphical temperature plot creation, as well. In other words, this device can also create a temperature data graph as a function of time based on the measurements, and based on a selected spot on the image. In some moments when a temperature is measured, also a thermal IR image is taken and stored.

US 8,240,912 ("Schreher") discloses a multi-zone non-contact spot thermometer, which measures temperatures with infrared spot detectors. There are two different measurement zones within a target scene, which differ in sizes between one another. It seems that no visible light image is combined with the IR images.

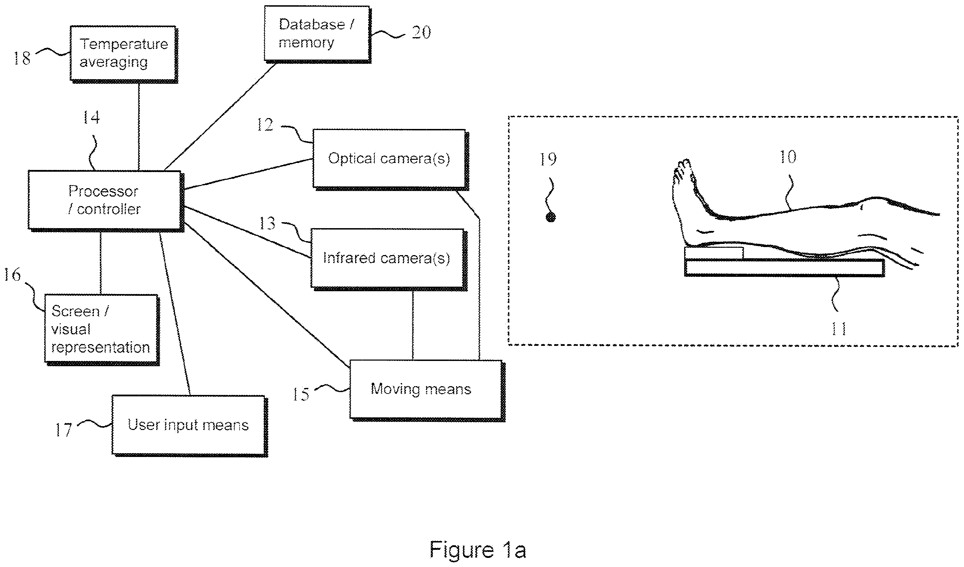

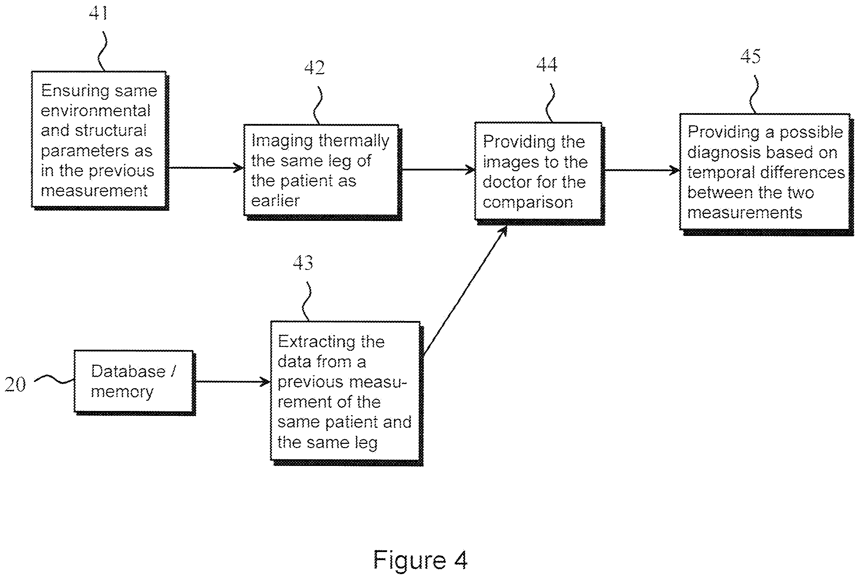

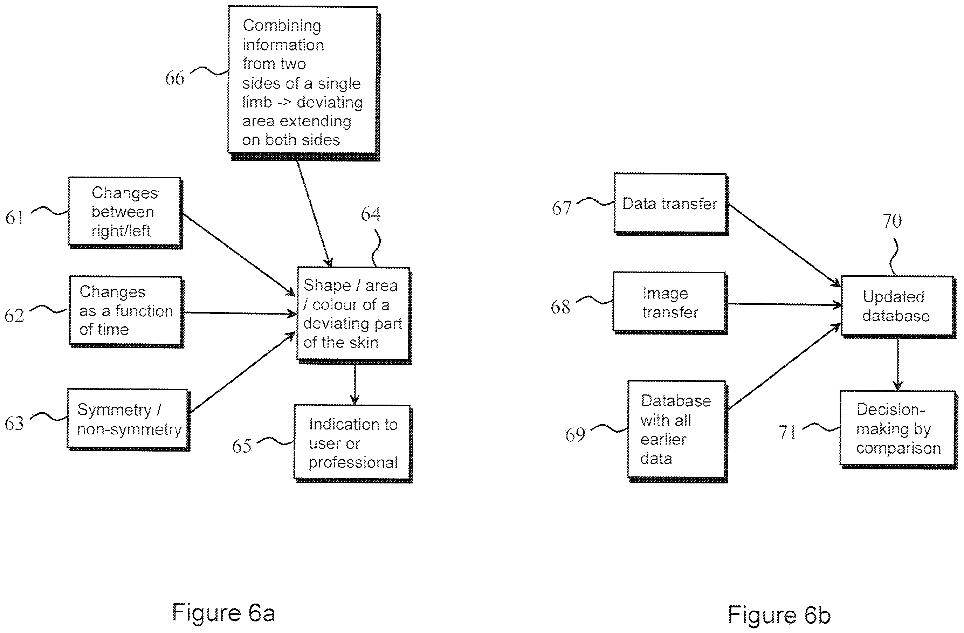

Regarding physical gantry systems and camera arrangements for self-monitoring of the legs, US 2016/0183879 ("Goldish") presents a system for screening skin condition on tissue damage. This can be used as a remote measurement device applicable e.g. at the home of a patient. Goldish presents a physical foot holder structure (see Figures 1A-B and 2-3) where a camera (like a smart phone) can be adjusted to take photographs of the feet of the patient, and the user may observe the images on a tablet or screen attached to the structure or by a separate computer. Goldish may be set to photograph the foot from different directions around it (Figures 4-5). The apparatus can be arranged for both feet. A manual operation is possible with a selfie stick kind of arrangement, and also the camera can be placed in the end of a flexible rubber-like stem for imaging the back of the patient. Both an optical and an IR image can be taken either separately or by adjacent corresponding cameras simultaneously. A microbolometer based thermal camera or a group of IR thermal sensors, i.r. infrared imagers, can be used. The IR image can be placed on top of the optical still image. There is mentioned a combinatory camera capable of taking and combining the optical photograph and the thermal IR image with a single device. The still photograph may reveal possible color changes present on the foot skin marking a possible tissue damage, and the rest enables the image to be taken from a non-pressed foot skin. So-called false positive results are eliminated in Goldish, which means that in order to determine a diabetic skin damage on a patient's skin, the optical camera must indicate a visible skin damage and the thermal camera must indicate a temperature difference in relation to the adjacent healthy skin within the skin damage area. Regarding the movements of the cameras, even a manual option is possible and a movement path obtained by a frame structure is shown in Figures 6A-B. One option is to move a single camera on a half-spherical track "270" either by a motor or manually by rotating a cable-driven camera with a crank. Stationary cameras "240a-d" can even be used. There is a supplementary camera "250" between the ankles of the patient where the direction of the camera can be pointed to two directions depending on which foot is imaged at the time. Finally, mirrors fixed in the support structure are shown in Figure 4 of Goldish.

US 2014/243683 ("Xiao") discloses a system, an apparatus and a method for human health evaluation utilizing Thermal Micro Texture (TMT) mapping technology. Xiao scans body areas of a patient with an IR camera, detects abnormalities in the body and analyzes them against information stored in a database. Results are returned to the patient in a pre-determined format. Xiao is about discovering a disease in an early stage by mapping and analyzing abnormal temperature changes in the body, and this can be used to prevent the disease from progressing from that early stage into a more severe condition.

As the main problem in prior art, there has not been presented any solution which would perform an accurate temperature measurement of the leg surface temperatures of the patients suffering for instance from diabetes. Furthermore, there has not been discussed a comprehensive analytics tool for the various temperature measurements utilizing previous measurements, or in wider sense, "big data". There is thus a clear need for an advanced method for giving accurate and helpful tools for the doctors in order to reveal some early signs of the problems within the feet or other body parts of patients.

### Summary of the invention

The present invention introduces an arrangement for imaging a leg or legs, or a desired part of them, of a human patient with an infrared imaging method, and especially obtaining surface temperatures along the skin of the leg, and enabling visual representation of the results. The (unclaimed) method can be implemented with a computer program, which is executed in a processor, such as in a regular PC connected with the measurement apparatus. A main target group for the present invention is the significantly large group of diabetic patients worldwide who suffer from various symptoms and side diseases and effects caused by diabetes. With the presented method and arrangement, accurate images showing the surface temperature distribution of the leg or the foot of a patient can be obtained, and the obtained images can be effectively provided under the inspection of a doctor for achieving a more accurate diagnosis for the current condition of the feet. The best diagnostic results are obtained by giving the taken thermal images of the leg(s) under human inspection made by a medical specialist, such as a doctor.

Besides the human leg, the invention could be applied also in imaging some other part of the human or animal tissue. For instance, the device could be used to image a human arm either fully or partially, or an animal foot or paw.

A secondary target group for the invention is formed by people using leg prosthetics equipment. Usually, this kind of usage results from an amputation process due to a severe injury or disease, and the end part of the remaining, living human leg tissue and its blood circulation needs to be monitored in order to prevent further damage in the human leg. The apparatus of the present invention is appropriate for such a measurement purpose as well.

The inventive concept comprises a system and a server with a software for diagnosing existence and progression of diseases of patients by imaging and mapping temperatures of human body parts, and analyzing differences between human body parts and changes in view of time. In this regard, we refer to the summarizing part in the end of the description, just before the advantages of the invention. A system in accordance with the invention is defined in claim 1. A server in accordance with the invention is defined in claim 9.

The computer program can control and execute appropriate method steps of the invention. The computer program may be stored in a dedicated medium suitable for this purpose.

### Brief description of the drawings

Figure 1a illustrates basic functional modules of the present invention, together with a part of a human leg, which is the object under examination,
Figure 1b illustrates an artificial intelligence (Al) system operating in a cloud service,
Figure 2 illustrates an example showing a human sole, which has been divided into eight sub-areas for analysis purposes,
Figure 3 illustrates an embodiment where both legs of a patient are imaged, and their results are compared between each other,
Figure 4 illustrates an embodiment, where a single leg of a patient is imaged, and the obtained image is compared to an earlier image taken from the same leg of the same patient,
Figure 5a illustrates an embodiment resulting to 3D modelling of an examined human part,
Figure 5b illustrates an embodiment revolving around automated analytics and big data,
Figure 6a illustrates an embodiment discussing area analysis between differentiating temperature areas,
Figure 6b illustrates an embodiment discussing the cognitively learning system, and
Figure 7 illustrates a foot sole where three different temperature areas are visualized during the foot analysis.

### Detailed description of the embodiments

Figure 1a illustrates different functional modules and physical elements, together with a human leg 10 lying on a support 11 which is the target of the examination. The support 11 can be shaped or formed in such a form and aligned in such an angle that the patient is able to rest his/her leg 10 in a fully relaxed position. Instead of a support, a resting platform or chamber can also be used in which the patient can rest on and place his/her leg in a horizontal position, or such a resting platform for the leg where the leg can be placed on so that the patient sits right next to the resting platform. The optical sensing means for visible light and for infrared light are essential in the invention, and these elements are an optical camera 12 and an infrared camera 13. In one embodiment, there can be several optical cameras 12 and/or several infrared cameras 13 movable in different locations around the examined leg 10. Furthermore, whether there are a single or a plurality of optical cameras 12, the optical camera or cameras 12 can be moved along a pre-specified route or routes. Also the IR camera or cameras 13 can be moved along a pre-specified route or routes, which in an embodiment are the same routes as the routes for optical camera(s) 12. The movements can be enabled in practice by moving means 15 which can be implemented by a physical route where the camera is able to move, such as a rail or a support or a gantry. This structure or apparatus is combined with a processor or a controller 14, which drives the cameras 12, 13 along the physical route in a controlled fashion. Alternatively, the cameras 12, 13 can be fixed to a controllable support arm which can be controlled like a robotic arm used in several manufacturing processes such as in car factories. The controllable support arm may comprise a telescopically formed arm where different arm sections can be placed as nested within one another. Furthermore, the controller 14 is able to control the alignment angle of the arm through a joint which is the connection point of the arm. Thus, in other words, the optical 12 and infrared 13 cameras are separately moved with corresponding arm structures around the limb or other part 10 of the patient, or the optical 12 and infrared 13 cameras are fixed together to a single arm structure in order to move them around the limb or other part 10 of the patient as a combined camera unit.

In general, the processor / controller 14 acts as a data analysis unit.

In an embodiment of the invention, the optical camera 12 can be controlled separately by the controller 14, compared to the controlling of the IR camera(s) 13. In an alternative solution, the optical camera 12 and the IR camera 13 can be placed side-by-side to a common arm structure where the movement of the combined camera structure can be driven by the controller 14.

In one embodiment of the invention, there are two different routes for the cameras 12, 13 where the two routes intersect orthogonally in front of the tip of the foot. An example of the location of the intersection point 19 is shown in Figure 1a. In other words, the first route can be a circular segment where the camera 12, 13 travels in a horizontal path around the leg 10 in such a manner that the mid-point of the route is the intersection point 19, locating along the axis formed by the shank, and in front of the skin of the sole. The route or the camera path may be circular but also an elliptical or other kind of a curved path is possible. The main principle is that the distance between the camera 12, 13 and the closest skin section of the leg 10 remains relatively constant during the orbital path of the camera 12, 13, but the distance does not need to be exactly constant during the camera rotation around the leg 10.

The second route can be created along a curved path which stays on a vertical plane where the second route can intersect with the first route orthogonally in the intersection point 19 in front of the middle of the sole. The second route can start in front of the calf skin and rotate vertically towards the intersection point, and further on top of the leg. Of course the second route may be travelled in an opposite direction, from the top to the calf side of the leg 10. Half of the route length may locate below the level of the leg 10 and also below the intersection point 19, and another half of the route locates on top of the level defined by the longitudinal axis of the leg 10, which is then also above the intersection point 19. Depending on how large section of the leg 10 is desired to be imaged, the end location of the curved route may locate directly above the knee or on some other location above the leg 10 between the ankle and the knee. The main principle in selecting the two routes is to have a line-of-sight visibility to all parts of the skin surface area of the leg 10 during at least some moment during the "scan", when the cameras travel along the two camera routes.

The support 11 shown in Figure 1a may of course physically restrict the line-of-sight view from the camera 12, 13 to certain leg surface locations pointing towards the support 11. One solution to this problem is to use a hanging strap suspended from a structure locating above the leg 10 where the hanging strap may support the calf approximately from the middle of the calf. Then the physical support 11 beneath the leg 10 or heel can be removed. Another option is to let the patient hold the leg 10 in a horizontal position while he/she stays in a sitting position, and to perform the camera movements during the stationary holding position of the leg 10. In case the patient is not able to maintain the leg 10 stationary in a horizontal position for a sufficiently long measurement time, one optional solution would be to hang the leg loosely to a downwards direction while the camera routes go around the leg 10 so that the intersection point 19 is right below the leg 10 and also below the sole. This requires special structure and camera moving means in a different positional arrangement where the patient is held above the movement structure during the examination.

When discussing the method steps associated with the two different routes or paths for the cameras, the following steps are performed:
- moving at least one optical camera 12 along a first path for taking a first set of optical images from the leg 10 or legs;
- moving at least one infrared camera 13 along the first path for taking a first set of IR images from the leg 10 or legs;
- moving the optical camera 12 along a second path for taking a second set of optical images from the leg 10 or legs; and
- moving the infrared camera 13 along a second path for taking a second set of IR images from the leg 10 or legs.

As a result of the image capturing steps with both the optical 12 and infrared cameras 13, the system obtains a three-dimensional model of the outer surface of the leg 10, as a result of optical imaging. With the infrared imaging, the system obtains received IR energies as a function of location. The human tissue radiates thermal energy based on the temperature difference between the surface spot of the leg skin and the temperature of the air surrounding the leg. The magnitude of this thermal energy can be measured through the IR detectors of the IR camera 13.

The varying amounts of received thermal energies can be transformed to a colored image, where different colours correspond to different amounts of received thermal energy. As a result, a 3-dimensional multi-color image is obtained.

As an alternative embodiment to the moving cameras presented above, the same operational effect can be created by having at least one mirror and stationary camera(s). In one embodiment, there is a single optical camera 12 and a single infrared camera 13. At least one mirror can be placed in predetermined location(s) and with controllable alignment angles, in order to reflect the visible light and the IR light to the corresponding camera(s). In an embodiment, the optical 12 or an IR camera 13 can be fixed in a stationary position, which can in one embodiment be along the axis defined by the shank and in front of the sole in spot 19 (with a line-of-sight to the sole). When a mirror is placed e.g. above the foot in a horizontal direction, it reflects the view from a desired area along the leg to the cameras 12, 13.

In one optional solution, the IR camera sensor and the visible camera sensor are placed next to one another, and the same group of mirrors (or a single mirror) is used to reflect both IR and visible light to the camera sensors. When the mirror angles are controllably changed and/or the mirror location(s) is/are moved, the "visible area" via the mirrors can be moved along the skin surface to be imaged; e.g. along the human leg. In one embodiment, the mirror angle is changed (i.e. with a rotational movement of a planar piece of a mirror) in a constant speed, or in a constant rotational speed around an axis.

In one embodiment, there can be implemented a combination of the above ideas; i.e. using controllable mirror or mirrors, and also controlling the movement of one of the cameras or many cameras or even all used cameras, simultaneously. The movements and the angles need then to be intelligently controlled so that the line-of-sight is obtained from all desired skin spots to be measured to the cameras.

In one embodiment, the material of the at least one mirror is selected from silver or aluminium. These materials have good reflection properties regarding IR radiation.

In an embodiment of the invention, the IR camera(s) 13 used in the measurements can be selected among common IR cameras already available in prior art.

A relevant characteristic about the environment where the measurements are performed is that the environmental and positional parameters regarding the arrangement (comprising also the relative positions between different elements of the system) need to be as constant as possible. This allows the repeatability of the measurements later as similarly as possible. When the conditions during the measurement situation are as constant as possible, it gives a possibility to compare the results between different measurement sessions with good quality. This in turn enhances the diagnostic possibilities and analysis results when the images are available to an examination made by a specialist, such as a doctor.

As a following step of the method, the first and second set of optical and IR images are combined into a 3-D color image showing temperatures and outer edges of the leg 10 or legs. This is in practice a three-dimensional image where dots with their coordinates along the skin surface of the leg and the surface temperatures in these dot locations are collected as a two-columned database, with a given resolution along the surface. This means that the accuracy can be separately defined, resulting in the number of rows required for the whole database.

For illustrative purposes, the database can be transformed to a 3-D color image displayable in a screen or other visual representation means 16. Different temperature values can be seen in different colours which can be tuned to change continuously as a function of location on the skin of the leg. The 3D color image can also be turned around and zoomed in/out by the user through the user input means 17. This can be implemented with the usual keyboard and mouse associated with a common PC.

A relevant feature of the software in connection with the displayed image is that the user may select a dot-like spot on the skin area of the imaged leg 10. The processor browses through the database and outputs the temperature value of the closest dot within the database, in one embodiment. In another embodiment, especially using less accurate resolutions (shown also as smaller databases), the processor may interpolate the temperature values of the closest spots in relation to the selected spot on the image. The resulting temperature value, whether a direct value or an interpolated value, is then output to the screen 16. The value may also be saved to a separate database.

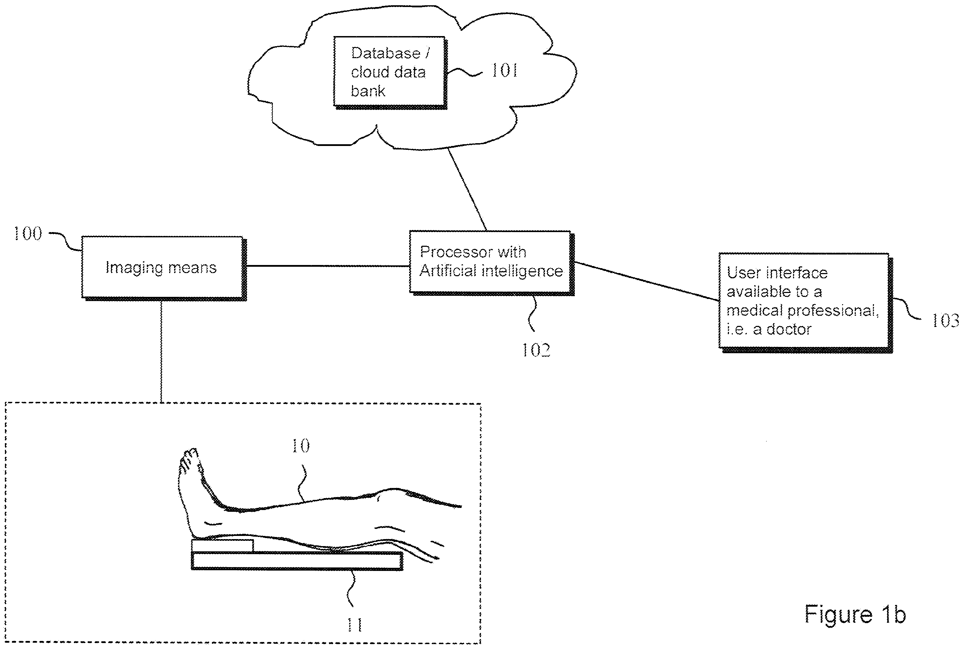

Figure 1b illustrates an artificial intelligence (Al) system operating in connection to a cloud service. The human body part to be imaged is the leg 10 resting on a support 11 in this example. The imaging means 100 comprises at least the thermal imaging means, i.e an IR camera or several IR cameras. It also comprises other imaging means such as an optical camera capable of taking photographs of the imaged area, or a laser scanning system. The imaging information is provided to be analyzed in a processor 102 which has artificial intelligence capabilities and access to cloud based information. Thus, processor 102 acts as a data analysis unit. A cloud data bank comprising related database(s) 101 is located in a cloud server or servers and it can exchange information with the processor 102. The data exchange between 101 and 102 allows cognitive updating of the cloud data bank based on newly taken measurements and deduced data based on that newly taken measurement. The Al algorithms can analyse patterns in view of other symmetrical part of the body or in view of an earlier thermal image of the same body part. The thermal measurement images and the deduced information based on the analysis work performed by Al algorithms (such as an early diagnosis estimate, or an alarm information) can be provided to a medical professional, such as a doctor. The doctor is able to check these pieces of information through an own user interface 103, such as a PC or tablet or any other suitable device (even smartphone). In this way, the medical professional may locate in different physical location than the examined patient. Also a different inspection time of the data is possible for the doctor compared to the measurement time of the patient, thus enabling a fluent analysis process and also a remotely available diagnosing arrangement for various diseases having temperature change symptoms.

The above concepts of Figure 1b are covered in more detail in the following process charts.

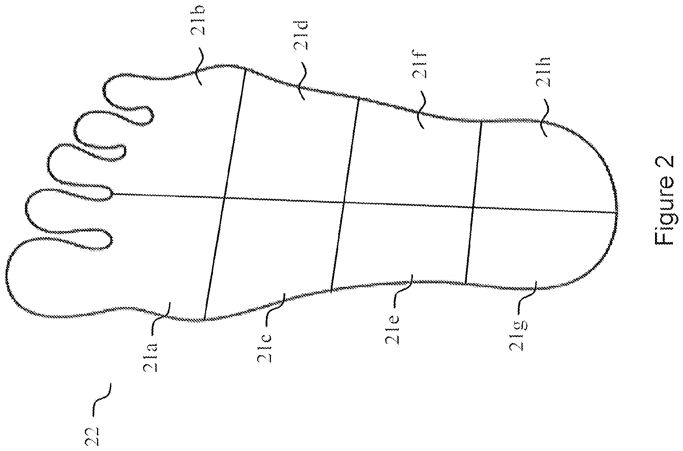

In another embodiment of the invention, the skin area of the leg can be divided to a number of sub-areas, and in this regard, also Figure 2 is referred. Here the sole 22 of the foot is shown from below and the sole area is divided into eight sub-areas 21a-h (also called as "virtual surface areas"). This is just an example of the area division and only a part of the whole leg to be divided into sub-areas. In the most common situation, the leg area which is covered by the optical and infrared cameras, is also divided in a selected number of sub-areas. The virtual grid formed by the sub-areas or the number of sub-areas can be selected beforehand by the user of the apparatus, and the selected grid lines can also be shown together with the above obtained 3D color image showing the actual leg. In other words, the division into the virtual surface areas 21a-h is configured to be based on a user selection among a group of predetermined virtual grids. When the user selects a desired sub-area from the whole skin area of the imaged leg, the processor 14 performs averaging for the temperature values 18 associated within the selected sub-area. The averaging can be calculated by taking into account all spots having a dedicated temperature value and locating within the selected area, summing all the temperature values (in Kelvins) together, and dividing the cumulative sum with the number of temperature spots within the selected sub-area. The result is thus an unweighed average of the temperature values within the selected sub-area. After the calculation of the average temperature within the selected sub-area, the averaged temperature result can be output to the screen 16, and additionally if desired, saved to the database as well. The system may be configured to automatically calculate the average temperatures for all sub-areas without any action of pointing by the user, and the system can after the calculation procedure save the average values to the database, for either providing the material for the diagnostic work made by the doctor, or to provide history data for future measurements and comparisons.

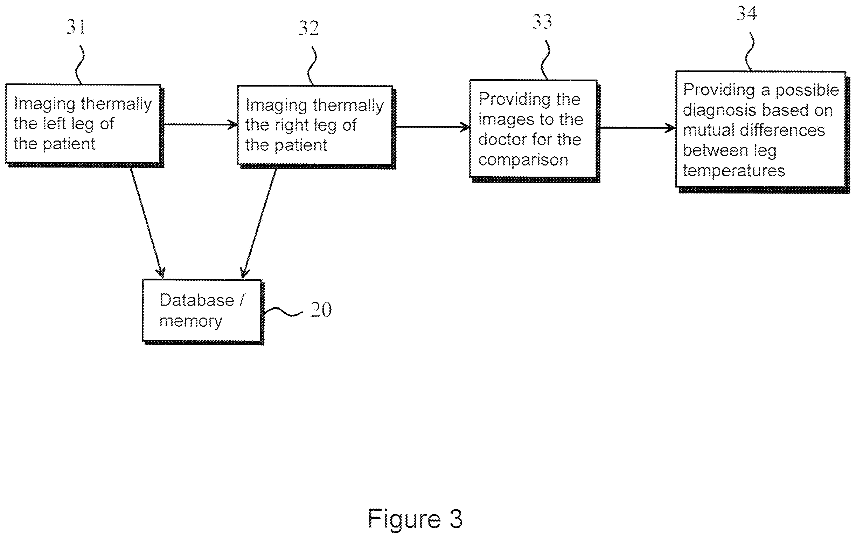

In an embodiment of the invention, the temperature measurement can be performed consecutively for both legs of the patient. This embodiment is illustrated as a flow chart in Figure 3. This is an important application area for diabetic patients especially but it presents a reasonable way to track differences between the patient's legs for also other diseases or injuries where there is a non-symmetrical temperature distribution between the right and the left leg. At a first stage of this embodiment, the left leg of the patient is imaged 31 according to the principle presented earlier. As described in connection with Figure 1a, a 3-dimensional image of the left leg is obtained, where the skin surface has been provided with an indicative colour depending on the respective temperature on each spot of the skin surface, the number of skin spots depending on the selected surface resolution. The thermal image of the left leg can be saved, and also the pairs of values, comprising the skin spot location coordinates and the corresponding temperature, can be saved to the database 20. After the measurement procedure for the left leg is completed, the same procedure is performed for the right leg. In other words, the right leg of the patient is imaged 32 according to the same principle as the left leg was imaged before that. The resulting image is correspondingly a 3-dimensional image of the right leg, and the observed temperature on the skin on the measurement moment can be seen with in different colours, similarly as earlier. Savings to the database or memory 20 are performed similarly as with the left leg.

The main step now in this embodiment is to output the two images, and/or the two sets of database values, and to provide them to the specialist 33 who may perform diagnostic activities based on the differences between the two legs of the patient, measured within the same examination session. The comparison work can be most easily performed with the colour images (either 3D-image provided on the computer screen), or as a group of 2D-images taken from the earlier calculated 3D-image. One option is to perform the measurements in a local health center or a municipal clinic, and the taken images can be transmitted electronically to a central hospital. In such a procedure, the patient does not need to travel to the central hospital, usually locating in a major provincial city, but the patient can be handled and imaged locally near his/her homeplace.

Based on mutual differences found between the leg temperatures in at least some area of the leg skin surface, the doctor or other specialist may provide a diagnosis 34, and also determine a severity of the diagnosis. This information can then be provided back to the patient in order to start proper treatment on the found disease or injury.

Figure 4 illustrates a flow chart, where a single leg of a patient is imaged, and the obtained image is compared to an earlier image taken from the same leg of the same patient. Of course, this procedure can be consecutively repeated for the other leg as well.

In this embodiment, the progressivity aspect of the diabetic symptoms is taken into account by taking the history data into account. This means that at least one previous imaging session has been made to the same patient, and for the same leg. These pieces of data are available in the database 20, accessible by the medical experts, such as the doctor of the patient. Let's say the previous measurement is the first temperature measurement for the diabetic patient A, made for the left leg, and it has been performed six months ago. Today, the patient arrives for the follow-up measurements. The patient will be set for the same conditions and arrangement as in the first measurement 41. This means that the support arrangement for the foot is preferably similar as earlier, and the room temperature is approximately the same as earlier. Also the patient is given instructions to perform similar activities (or to avoid certain actions or exercises) right before the imaging session, as was the case before the first measurement session six months ago. The left leg is then imaged 42 the same way as it was measured in the previous measurement session six months ago. When the thermal imaging has been performed according to the method and apparatus disclosed above, the images from two different measurement sessions are provided to the doctor for the comparison 44. In other words, the latest image of the left leg is provided to the doctor, and furthermore, the extracted measurement data from the previous measurement 43 is output for the doctor as well. In practice, this can be implemented as showing two images in parallel arrangement within the same screen, the left leg image six months ago, and the left leg image now. Thereafter, this provides an easier way to make a diagnosis based on the temporal changes in the condition of the leg 45. In case, there is a sub-area of the leg, which is significantly colder than six months ago, it is an alarming condition, which the doctor can address with an appropriate diagnosis and treatment for the patient.

In other words, the above embodiment can be described in a summarized way like this. The arrangement is configured to adapt same environmental and structural parameters as in a previous measurement session of a given limb or other part 10, to measure the given limb or part 10 of the patient again, to extract the previous measurement session data from the database 20 concerning the same patient and the given limb or part 10, and to provide the two images of the given limb or part 10 to a specialist for diagnostic purposes, in order to see temporal changes in the measured limb or part 10.

The time period lengths are merely examples, and they may be selected freely according to the severity of the situation with the patient, or according to medical guidelines for a certain medical condition, or according to law or according to other criteria selected by the doctor.

In one embodiment, the history data of the patient's leg(s) can be used in even greater extent, by e.g. providing two previous measurements as history data for the comparison with the situation now. This means that three parallel images can be provided for the doctor, showing history data and the trend in diabetic feet condition for 1 year ago, 6 months ago and now. Such a measurement principle allows to see whether the condition has deteriorated in a slower speed or in a more rapid speed than 6 months ago. Of course, this allows performing a more long-term analysis, if even more sessions of previous measurements are taken into account.

In one embodiment, not just a single leg is measured for obtaining temporal changes in a given time period, but both legs can be consecutively measured in order to make a complete check for both feet of the patient.

For more severe diabetes, a measurement cycle of 3 months can be applied for the measurements discussed above. Of course, any other measurement cycle length or measurement instant may be selected based on the situation of a patient, or based on a decision made by a medical expert.

Now back to the general method for temperature imaging of a leg, the result may be a temperature map illustrating absolute temperatures on the map showing the leg. The map may be a three-dimensional image which can be rotated and zoomed and otherwise handled in a computer screen. Of course it is possible to extract two-dimensional images from certain sections of the leg, e.g. showing only the sole surface if that is the interesting part in the measurement.

The averaged temperatures and the grid idea (various sub-areas) can be implemented with also the embodiment discussed in Figure 3, but also with the embodiment discussed in Figure 4. Therefore, the software may output images of both legs where the average temperature of each sub-area is calculated and shown on the screen. Furthermore, the same can be applied for the historical image of the leg, and for the image taken in the current measurement session. This kind of temperature output gives direct information even for non-specialists. If there are e.g. eight sub-areas covering the sole of the foot, two parallel images with eight parallel temperature values allow easy comparison of the values without having to select any dedicated spot on the images. Of course, more accurate temperature information can be obtained through selecting a dedicated spot on the current measurement image, or on a certain previous image taken from the database.

As we discuss the 3-D color image as a way to show different temperatures of the leg skin, alternatively there might be a grey-scale image showing those temperature differences in the changing darkness of the grey color (from black to white, with a given number of intermediate grey tones). Furthermore, there might be a different way to show different temperature areas, such as different line, shade or pattern additions placed on the taken image, made with image processing tools.

As a different optional solution to imaging the limbs, there can be used an intermediate material around the limb which can be removed from the limb after a certain period, and imaged later. By this we mean a wearable sock or fabric which can be manufactured from material which will absorb heat well. The sock or fabric is wrapped around the leg for a given time period. The heat from the leg will transfer and absorb into the material of the sock or fabric. After the "absorption period", the sock or fabric can be taken off. After this step, the removed sock or fabric is opened and placed on a planar surface. From the seams of the sock or fabric, the system will know the correspondence between the planar coordinates of the sock or fabric, in relation to the spot on the human skin. By imaging the planar sock or fabric with a single IR camera, the temperature image in the coordinates of the human leg skin can be obtained. Otherwise, the other details of the invention from the earlier description is applicable to this embodiment as well. If the material is chosen appropriately for the sock or fabric, the image quality is good for this embodiment as well.

In other words, the two inventive ideas have common features and principles behind them but the first concept measures the 3-dimensional leg directly while the second concept measures the two-dimensional, opened sock or fabric after it has been removed from the 3-dimensional leg.

Generally speaking about the saving of the obtained images, the control means 14 is configured to save the created combined images in either image form, or in numeral form comprising coordinates and corresponding temperatures, to a database 20.

Back to the general inventive idea, no matter whether an intermediate material is used or not, the obtained temperature information can be handled with various ways. In one embodiment, the measurement results are saved into cloud. Furthermore, the system can be made to a cognitive system where temperature data and possibly also other kinds of measurement results are all available in the cloud. Furthermore, when the current measurement data is saved into the cloud, this increases the saved measurement data in the cognitive system. The saved measurement data comprises the history data of the given patient, but it may also mean history data of all saved patient (for a group of various different patients) measurements or a predetermined part of it. The whole big data of all gathered information may be classified e.g. based on different severity levels of diseases. This can mean that different leg temperature measurements can be classified with severity levels such as "everything is ok", "mild diabetic symptoms", "severe diabetic symptoms". Also the data can be classified based on patient characteristics, e.g. based on age or gender of the patient. Alternatively, it is possible to link with each measured image of the leg, a given diagnosis, which is saved in connection with the image. When a large number of images are available as reference images, the newly taken image of the patient can be used in the diagnosis which could even be made without a human specialist, meaning artificial intelligence. In other words, the previously measured data is linked with a given diagnosis associated with the relevant measurement, and this information acts as a reference tool for the diagnosis in view of the current measurement. Of course, the reliability of the diagnosing process can be enhanced if the real doctor observes the images manually but the doctor could use the diagnosis given by the "machine" as a helping tool. With the cloud based artificial intelligence diagnosing system, it would also enable a remote health service, where the patient takes the image even by him/herself, sends the image to the health service provider having access to the cloud information, and the system uses the analytics and it sends the diagnosis (e.g. "severe diabetes" / "mild diabetes" / "risk for diabetes" / "no diabetes") back to the patient without any human doctor taking part of this diagnostic work. This is an intriguing future development scenario for effectively diagnosing diabetes of a remote patient.

Next we discuss supplementary concepts present within the same inventive idea as already discussed above.

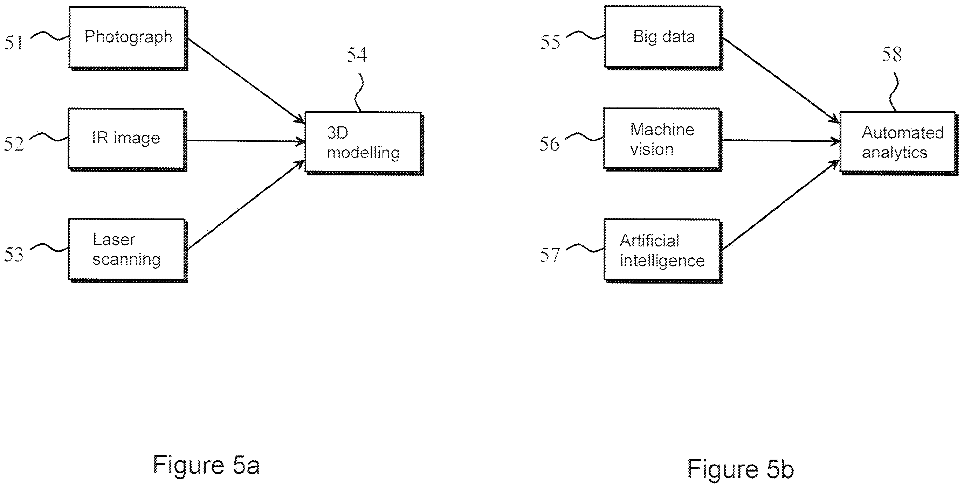

The first supplementary concept is adding three-dimensional (3D) modelling as a further procedure after all the appropriate and desired imaging have been taken from the examined part of the human body. Figure 5a illustrates these functional steps and elements as a simplified flow chart. Optical images e.g. still photographs 51 are taken from different sides of the examined human part. In this embodiment, no physical gantry is emphasized; instead the photographs are taken from different sides of e.g. the foot so that the whole skin surface of the foot is visible in at least one of the taken photograph. The same principle applies in the thermal images, i.e. IR images 52, taken by at least one infrared camera. A further option is to use laser scanning 53 for obtaining three-dimensional scanning of a human body part. The laser scanning arrangement can be set to move around the examined limb, for instance, and scan the whole surface of the limb. This can be achieved by moving a single or several laser scanning modules on top of the examined surface area.

A further option is to use magnetic resonance imaging (MRI) for obtaining 3-D tissue images of the examined part of the human body. MRI images can be taken from any section of the human patient but the usual areas to be imaged with this technique is lower back / hip area, the cervical spine in the neck, and the brain but also limbs can be MRI imaged.

A further embodiment may include some other kind of imaging of a desired part of the human body, e.g. obtained by X-ray imaging, in helping to create a 3D-model of the desired part.

The purpose in the 3D-modelling is to use at least the thermal (IR) image 52, and possible additional images (optical photograph 51, MRI image, X-ray image) in order to have sufficient information from the skin and within the tissue of the desired part of the human body in order to create a 3D-model for further analysis or educational purposes. It can be said that mapping of temperatures is performed on the measured IR image so that the correspondence between the location in a 3-D modelled human body part, and its measured temperature is created. Additionally, blood vessels visible from the skin can be taken into account from an optically obtained photograph of the tissue. In an embodiment, the thermal and optical image information is used as a source information for 3D-modelling.

A second supplementary concept is to add the concept of the big data into the use of the inventive arrangement. Figure 5b illustrates the main parts of the cognitive system which results in automated analytics 58 of the thermal measurement results of the part of the human body. Big data 55 means all available data in the cloud and for the inventive concept, this means all previously obtained thermal images of the human body part. In other words, thermal image information can be picked from a cloud data bank comprising information of all available patients. The image information can be linked with a diagnosed disease information, or progress information of a given disease. While the disease or progress information has been given by the doctor for the earlier taken images or sequence of images, such data is later available as part of "big data".

Machine vision 56 arrangement can implement the automated imaging of the examined part of the human body. This applies to both optical photographs and thermal IR images.

Artificial intelligence 57 algorithms (one or several) can be used to detect interesting parts of the thermal images. For instance, if there is a large thermal gradient in certain part of the image, the algorithm will detect such area, determine its borders and it may calculate the surface area of the skin with a different temperature as the surrounding tissues. These algorithms can be used in the already taken thermal image bank in the cloud and combined with the doctor's diagnosis. In this way, the Al algorithm can learn the correspondence between different thermal areas in a limb with a given disease of state. Also the algorithm may learn from the correspondence between thermal gradients (thermal change magnitude as a function of surface distance) and given diagnosis by the doctor. Also the differences in the sides (left vs. right limb) or as a function of time (previous measurement vs. current measurement) are good piece of information for helping the Al algorithm to improve with the automated analytics 58. The automated analytics 58 may output the state of health or an alarm signal for the examining doctor, or even a machine-given diagnostic parameter for the user or professional.

The artificial intelligence 57 algorithms may use pattern recognition algorithms where the found patterns are classified. The Al algorithms may also take the type or severity of the disease, the patient's age, the patient's weight into analysis as well, in order to determine the severity of the found thermal deviation in the patient's body. This applies especially to diabetic feet examination.

We then discuss the main analysis options for the obtained thermal image. In this regard we refer to Figure 6a.

A first analysis option is to compare a thermal image of a right side of a human patient to a thermal of a corresponding left side of the human patient 61. The imaged parts can be left and right feet, or left and right hands. The imaged part can be a left and right side of a given part of a human torso. The imaged part can be any part, where symmetry exists between two areas of the human body. The inventive concept can be realized by taking subsequent thermal images of the symmetrical parts, so that e.g. the left foot is imaged first and the right foot is image after that. The analysis unit, e.g. a controller or the control means, is configured to compare the left side thermal image with the right side thermal image and to detect any differences between them. The difference can be a different thermal area distribution between the two thermal images. For instance, a lower temperature area on a left foot may be of different surface area or shape compared to the lower tempereature area on a right foot. A spot-like temperature value can be different between the right and left foot, for instance. Alternatively, an average temperature within a specific predetermined area of a left foot can be noticeably different than within a corresponding predetermined area of a right foot.

A second analysis option is to monitor the changes of the imaged part of the human body as a function of time 62. The currently taken thermal image can be compared with the previously taken thermal image, or to an even earlier taken thermal image. The time frame between two consecutive imaging sessions for a single patient is dependent on the examined disease, and determined by a doctor or other specialist in the most usual case. The second analysis option is independent of the available time frame, meaning the any earlier taken image of the same patient and the same part of the patient's body can be observed together with the currently taken (or most recently) image. Of course, the progress of the human condition and the current progress rate of a disease may be examined by using the current thermal image and any number of earlier taken thermal images. The used technique can be overlapping the thermal images in top of one another to find differences between them. Machine vision algorithms can be applied in detecting the changes in subsequently taken images. The result in the second analysis option 62 can be obtained by comparing two previously obtained images with one another, and giving a health status parameter for the change. The health status parameter can be "unchanged/stable", "worsened", "significantly worsened", "improved" or "significantly improved". The health status parameter is naturally linked with the observed disease, associated with the patient from the earlier taken image or images. The status "alarming" can also be given to the image if it deviates from a "normal" healthy situation by a certain threshold value. Such an image is then submitted under supervision of a medical professional. Of course, the image with any health status parameter can be submitted to a doctor or other medical practitioner for further observation and analysis 65.

Generally, the health status parameter can be a piece of diagnostic information concerning any desired disease, malfunction in the human body, or operation in the human body. The health status parameter may comprise a qualitative estimate on the patient's health (i.e. the severity estimate of the diagnosed disease) but this is not necessary. Any piece of information estimating the current situation of the patient's health is sufficient for the health status parameter. In this context, the main purpose is diagnosing existence and progression of diseases of patients.

A third analysis option is to observe possible symmetry 63 between the images of first and second thermal images where the imaged parts can be in symmetrical parts of a human body. For instance, an area with a thermally deviating sub-area in a left foot can be observed against a thermally deviated sub-area in a right foot. The outer edges of the first sub-area can be compared with the outer edges of the second sub-area, and if there is a change in the shape or size of the sub-areas, the controller of the arrangement records this. In this way, symmetrical sections and non-symmetries between observed images are examined and found.

An additional option is to take a thermal image from two, opposite sides of a single part of a human body, like the sole of the foot and the top side of the foot. The images together cover a combined surface of the skin which cannot be imaged with a single IR camera location. The information obtained from two opposite sides of the human body part (such as limb) are combined 66 and possible single thermally deviation areas extending on both sides of the human body part are detected. In this way, larger thermally deviating areas can be tracked. Also effects, which extend to different sides of a single limb, can be tracked this way, and it improves the diagnostical decision making in diseases which have effect in broader areas of the human tissues. The information in two opposite sides of the limb can be combined with analysis options of 61, 62 and/or 63 in order to analyse a larger area, concerning the shape, area, colour or non-symmetry in comparison to a reference image.

The steps of the analysis options 61, 62, 63 and 66, and further resulting into step 64 can be implemented with one or several artificial intelligence algorithms implemented in a local or cloud based computer or server (like e.g. processor 14)

As a result from analysing changes between any two images from the steps 61, 62 and/or 63, and also taking the possible larger thermally deviating area from step 66 into account, the controller determines the current status of the deviating part of the skin and also the change magnitude compared to the reference image as discussed in steps 61-63. A distinctive shape of a thermally deviating area, or a notably changed area of a thermally deviating tissue or a deviating colour in the imaged area is the resulting interesting parameter or a group of parameters 64, which can be indicated to the user him/herself or to a medical professional 65 such as physiotherapist, doctor or a nurse. Al algorithms give the possibility to automize the search against a larger number of reference thermal images available in the cloud data bank (i.e. the updatable database). The system is a learning system because the thermal image bank together with the deduced diagnosis data increases all the time during the use of the invented measurement and analysis system. Also external thermal images achieved from other sources may be used in updating the cloud data bank. Furthermore, Al algorithms will improve as a function of time as such because the calculation power of processors will increase over time. This ensures even better results, and this leads into a system comprising deep learning capabilities, resulting in a cognitive and highly progressive system.

In the embodiment comprising a wearable sock or fabric wrapped around the examined part of the human body, such as foot, the images will be created a bit differently. Thermal energy from the examined part of the human body (like foot) absorbs to the sock during the time when the sock is worn to the patient's foot. The thermal energy will transfer from the human tissues to the sock until when the temperature of the skin corresponds to the touching part of the sock in each skin location wrapped by the sock. This requires a certain period of time to settle. When that time period is reached, the sock can be opened, and the thermal camera takes an IR image of the planarly placed sock or fabric. In a regular situation, the IR image is taken in directly orthogonal direction from the planar fabric; e.g. with the IR camera pointing directly downwards towards a fabric laying on a horizontal plane, e.g. on a table. The thermally deviating areas can now be inspected from a planar, thermal 2-D image. No matter whether the thermally deviating areas locate on opposite sides of the foot (sole and top), the planar image will reveal the total size and shape of such areas. In the processor / controller 14, there must a calculation algorithm which makes the correspondence between the spots of the 3-D human part outer shape and the 2-D planar image coordinates. In this way the planar image information regarding the temperatures can be converted to temperatures of a 3-D image of an actual foot. In practice this means the same as the mapping of temperatures. This information can further be used in 3-D modelling of the examined foot (or any other human part). Generally, it is of course important to take the thermal image of the planar fabric quickly after the fabric has been taken out from the patient. The obtained thermal information using the fabric or sock can be combined with e.g. an optical photograph from the foot itself, in order to add visual inspection information e.g. regarding skin colours to the analysis. Also other options disclosed in the application are similarly applicable to the sock embodiment as well.

Figure 6b illustrates an embodiment discussing a cognitively learning system associated with the obtained image data. Data transfer 67 means all data and deduced information based on the taken thermal image and possible other images such as an optically obtained photograph. All the image information is transferred 68 as well to a database. The data of step 67 can be temperature value information as a function of coordinates in a form of a data table, an averaged temperature value within each sub-area in a form of a data table. The deduced data can be disease information or progress / status information of a disease based on a taken image. The deduced pieces of information are linked as auxiliary data for the given image. Thus, steps 67-68 form all information obtained from the currently taken image, while all the earlier obtained and saved corresponding images and their auxiliary data is marked as "Database with all earlier data 69". Step 70 marks the cognitive nature of the image management system where the newly obtained image with all its deduced information and auxiliary information are added to the database. This process increases the amount of reference data in recognizing different diseases and their progress when a new image is taken from a patient. This relates essentially to steps 55-58 of Figure 5b as well. The updated database 70 means that the amount of "big data" increases as a function of operating time, resulting in a cognitive health status determination system based on thermal imaging. In other words, the system is a learning system because the measurement and diagnosis information available to the analysis at hand will increase during the use of the presented system and method. Step 71 is making a health status decision by performing a comparison of the current image and any reference image(s); thus, this step is in line with step 64 of Figure 6a. It can be said that over time the decision-making 71 will become more accurate, even for the automated analytics tools such as machine vision systems.

In general, the described system can be set to determine a normal temperature range associated with healthy human "patients". For instance, it can be determined that a normal temperature range of a human foot is between 22 ... 32 degrees, and such data is used as reference data for a newly taken thermal image. In addition to deviating sub-areas when considering temperatures, also hot spots and cold spots can be recognised from the taken image and marked as auxiliary parameters.

In one embodiment of the invention, different coloured areas can be selected into the examination from the obtained image. The areas of each coloured areas can be calculated by the system, e.g. the area of the warmest area (e.g. in red) and the area in the second-warmest area (e.g. in violet) and the area of the thirdwarmest area (e.g. in yellow). Such calculated surface areas can be compared to the same parameters obtained from an earlier measurement.

In a further embodiment of the invention, a first image is taken from a first side of the part of the patient, such as from the bottom of the foot sole. After that a second image is then taken from an opposite side of the part of the patient, such as from the top of the foot. By this it is meant to take a larger image from the skin surface where the two areas are adjacent but only visible from opposite sides of the imaged part. If there is a larger thermally deviating area (like cold area or a group of colder spots) within the larger image, it can be used in the health parameter determination. Also symmetries between the thermal larger image of a left foot can be checked against the thermal larger image of a right foot, and if the thermally deviating area is not symmetrical with the corresponding reference image, an alarm can be set for the examining professional for this image.

The diseases, whose progress or existence may be detected with the present invention, are various. Diabetes is one of the most prominent diseases to affect in various ways and also in various parts of the human body. Diabetes causes malfunctioning and damages to the peripheral nerves, in other words, disorders falling under diabetic neuropathy. Diabetes causes many effects to the feet if progressing to a certain level; the symptoms may be diabetic foot ulcers or numbness based on damages present in the peripheral nerve system. There can be present a peripheral artery disease ("PAD") which with diabetic patients is called as diabetic angiopathy. This has a main symptom of causing poor blood circulation to the extremities of the human body, such as feet. This in turn causes decrease in the temperatures of the feet tissue and it can be sensed with thermal imaging as disclosed earlier in the disclosure of the present invention. Also the decreased temperature area and shape and extent in different sides of a single foot are important in determining proper health situation or diagnostic result.

The analysis of the thermal image can be combined with visual skin analysis. The skin of a diabetic patient may comprise calluses. Also foot ulcers are a severe symptom of diabetic feet. By analysing thermally the area on and near a callus, important information can be obtained for determining the status and progress rate of a diabetic patient. The thermal image gives valuable information on what kind of damage or what kind of tissue "hides" behind a callus; the system according to the invention thus gives valuable information for the evaluating medical professional. Furthermore, the locations of the calluses on the skin may be interesting for the evaluating doctor in his/her further analysis work.

Generally, peripheral neuropathy due to any other cause than diabetes, is also a significant group of diseases or damages which can be tracked with the imaging system according to the invention.

Another types of diseases are Charcot related diseases which can be well examined with the presented system according to the invention. An example of these diseases is neuropathic arthropathy, i.e. Charcot joint disease, which is characterized by swelling or even deformities of human tissue, based on a degeneration process of a weight bearing joint. In even worse situation, the bone material may even collapse due to destruction of the bone material. Also increased skin temperature (e.g. 3-7 degrees) is a characteristic symptom near the joint. The presented imaging system is well suited to discover these symptoms, by also utilizing optical photographs revealing the swelling of the tissue. The present invention is well suitable for observing improvement / healing process from a Charcot related disease or symptom as a function of time.

One more disease group to benefit from this method is rheumotoid arthritis and similar, which causes joint inflammation and thus temperature rise on the skin surface around inflammated joints. All of the above mentioned methods and arrangements can be applied to diagnose arthritic joints and also other diseases which cause local inflammation in joints or other body parts, such as erysipelas, Raynaud syndrome, peripheral arterial block etc.

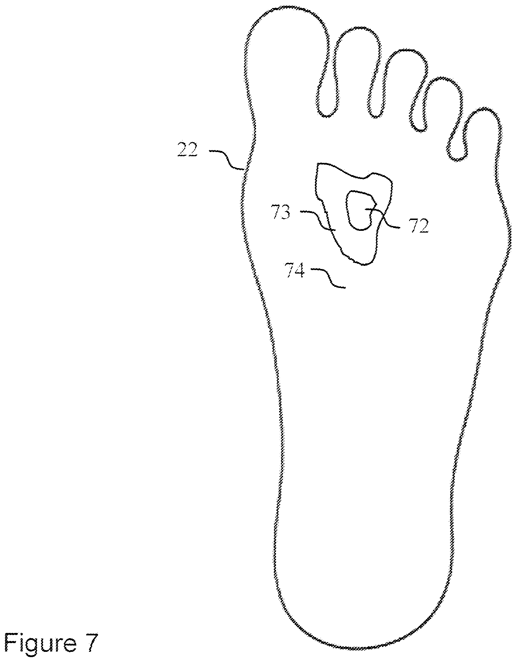

Figure 7 illustrates a foot sole 22 where three different temperature ranges are marked with three skin surface sub-areas 72, 73 and 74. The innermost sub-area 72 marks the lowest temperature range, e.g. 18-22 °C. The next sub-area 73 may mark the intermediate temperatures, e.g. the range 22-27 °C. The rest of the skin surface forms the largest sub-area 74, staying in temperature range 27-32 °C. The temperature values are merely exemplary. The interesting parameters are the cold spot location and temperature in sub-area 72, the average temperature of sub-area 72 or 73 or the average temperature of their combined area, the thermal gradient, i.e. the rate of the temperature changes as a function of distance (in this example, the maximum gradient locates just right from the sub-area 72), the shape of the thermally deviating area (in this example, the outer edge of 73). In an embodiment, the obtained (deduced) parameters from the thermal image are linked with the image, and the resulting pieces of information is compared to the earlier obtained image or images and their deduced parameters. Thus, a health related parameter is obtained as a result. The health related parameter may be a intermediate result or a starting estimate of the condition of the patient for the doctor. The doctor is then able to proceed further with the diagnosis and treatment by excluding non-relevant and wrong diagnosis with further actions made by the doctor. Thus, the present invention is an assistance tool for easening the workload required by the doctor by giving a helpful estimate on the condition and the progress of the disease carried by the patient.

The obtained thermal images can be visualized in a software tool comprising various selectable parameters. Depending on the temperature range of the obtained, measured part of the human body, e.g. foot, the software tool gives the user the possibility to select the minimum and maximum temperature values of the dynamical range. The selected dynamical range may comprise a predetermined number of colours for visualizing effectively the selected dynamical range. Because in some diseases the temperatures may be higher than average healthy tissue, and in some diseases lower than average healthy tissue, the dynamical range is freely selectable by the user.

In one embodiment, the system is configured to save the temperature values of each pixel of an interesting area into a database. For the information saved this way to the database, a maximum, a minimum, or average values, or the distibution of values may be extracted in order to analyse the thermal values of the interesting area.

Generally, a steep thermal gradient in the human tissue indicates a nerve-based damage (i.e. neuropathy) in the body. A less steep thermal gradient however indicates a possible problem relating to blood vessels of the patient.

Generally, the mapping of temperatures means making a correspondence between measured temperatures and their source location on the human skin, no matter whether the human body part is directly thermally imaged by an IR camera or cameras, or whether the IR camera is set to image a planar sock or fabric after its heat absorption from the human skin.

It can be summarized that the present invention combines a clinical thermal imaging with possible other imaging, and performs intelligent analytics on the obtained image and parameter information. Machine based tools and artificial intelligence may be used for analysing the obtained image information against the information already part of the big data.

A further option is to use the obtained thermal and other information on the imaged part of the human body as input data for 3D modelling tools. In this way, a 3D model of an interesting part of the human body can be created. For instance, the result can be a 3D model of the human foot, which can further be used in educational or diagnostical purposes. A 3D modelling tool may be a separate entity, and the system according to the invention may transfer the obtained imaging information to the 3D modelling tool.

The inventive idea comprises thus a corresponding method, and a server with a software which are in line with the correspondingly presented system for diagnosing existence and progression of diseases of patients. The server comprises or has access to needed memory units capable to store the required information, i.e. the cloud data bank information. A cloud based processor and/or a local PC processor, and a UI unit available for the medical professional are also useful parts of the inventive concept in an embodiment of the invention.

Generally, the data analysis unit is an element which processes the information, e.g. the processor or controller having access to the measurement data obtained from the patient and also to the data in the cloud (big data).

All features and physical elements discussed in connection with the system can be implemented in the corresponding method and server with a software where applicable. Similarly, all features and steps discussed in connection with the method can be implemented in the corresponding system and server with a software where applicable.

Advantages of the invention comprise the following:
- The arrangement provides accurate and helpful tools for the doctors or other specialists in order to reveal some early signs of the problems present in the feet of diabetic patients. This further enables quick treatment possibilities for the patient, and proper treatment can be addressed much quicker compared to feet inspection methods in the prior art.
- The measurement method is reliable, repeatable, accurate, quick to perform, and a non-harmful method because there is not any X-ray radiation involved.
- The results can be saved and easily compared with other measurement made at a different time.
- The doctor does not need to be present in the same premises where the measurement takes place. The analysis may also be performed later after the actual measurement situation.
- The software interface provides useful and easy selection of desired places and areas along the leg skin.
- The imaging method as such is not manually performed, and this removes the errors caused by subjective analysis based on the nurse touching manually the feet of the patient.
- Big data and cognitive nature of the system and the database makes the presented system as an improving system over time. Analytics comprises many options based on symmetry, changes as a function of time, thermal gradient magnitudes, and the system benefits also from the earlier obtained images and their related health parameters and the earlier obtained progress information of the diseases.
- The present invention is not restricted to any presented gantry system. The thermal and possible optical images can be taken with any moving, manual or automized system, if the whole interesting area is imaged.
- The tunable dynamical range of the thermal image gives illustrative results, no matter whether the symptom is a hot spot or a cold spot or area, correspondingly.
- The system is a learning system, where new measurement data, deduced data related to the new measurements, and diagnosis data made by a medical professional in view of a given measurement of the patient can be added to the cloud, increasing the amount of "big data" available in later measurements.

The present invention is not restricted just to the examples disclosed above but the invention is defined by the scope of the claims. In case some characteristic parts or details mentioned above (even in two separate embodiments) are interconnectable with one another, this combination may be done in order to create an alternative embodiment of the invention.

## Claims

1. A system for diagnosing existence and progression of a disease of patients by imaging and mapping temperatures of human body parts, and analyzing differences between human body parts and changes in view of time, wherein the system comprises:
- thermal imaging means (13) configured to capture at least one thermal image (52) of a human body part from at least one direction, comprising temperature values in a predetermined area of the human body part, wherein the thermal imaging means is an infrared camera,
- a data analysis unit (14, 102) receiving and analyzing thermal image information (52) of the thermal image, wherein the data analysis unit (14, 102) is further configured to
- compare the thermal image information (52) to at least one reference image information using artificial intelligence algorithm or algorithms (57), where the reference image information is either a previously taken thermal image of the same human body part of the patient (43), a thermal image of a correspondingly symmetrical part of the human body part of the patient (31, 32, 61), and/or thermal image information picked from a cloud data bank (55, 69, 101) comprising information of all available patients,
**characterized in that** the data analysis unit (14, 102) is further configured to:
- merge an optical image (51) of the human body part into an analysis performed by the data analysis unit (14, 102), where the optical image (51) is obtained by a regular camera (12) or by laser scanning,
- perform pattern recognition and pattern classification on the obtained images by the artificial intelligence algorithm or algorithms (57), taking the type or severity of the disease, the patient's age and the patient's weight into analysis to determine a severity of a found thermal deviation in the patient's body; and
- give a health status parameter based on the performed comparison and the merged optical image (51), where the health status parameter is a diabetic progression information or a Charcot based disease status information.

2. The system according to claim 1, **characterized in that** the data analysis unit (14, 102) is further configured to pick cold spots, hot spots, thermally deviating areas, their temperatures, area sizes and their shapes, their non-symmetries and/or thermal gradients around the thermally deviating areas into the analysis.

3. The system according to claim 1, **characterized in that** the data analysis unit (14, 102) is configured to add a deviating colour information from the optical image and/or possible outer surface deformations into the analysis.

4. The system according to any of claims 1 - 3, **characterized in that** the system is a learning system, and the system is configured to cognitively update the cloud data bank (55, 69, 101) by adding the taken thermal image information (52) together with its deduced parameters, and/or health status determination result given by a doctor into the cloud data bank (55, 69, 101), as part of the learning system.

5. The system according to any of claims 1 - 4, **characterized in that** the system is configured to take subsequent thermal images on two opposite sides of the human body part, and to combine these images (66) in order to reveal a possible larger thermally deviating area in the human body part.

6. The system according to any of claims 1 - 5, **characterized in that** the system is a learning system, and the thermal image information (52) comprising pixels and their temperature values, and deduced health status parameter obtained by the system, and possibly manually updated health status information by an examining doctor, are configured to be added into a data table forming the thermal image information (52) in a cloud data bank (55, 69, 101), as part of the learning system.

7. The system according to any of claims 1 - 6, **characterized in that** the system comprises a user interface (17, 103) where the user is able to select the dynamical range comprising minimum and maximum temperatures, and a display (16) is configured to illustrate the temperatures of the imaged part of the human body with a range of colours alternating within the selected dynamical range.

8. The system according to any of claims 1 - 7, **characterized in that** the temperatures of the human body part are configured to be measured with a sock or fabric placed or wrapped around the body part to be measured, and after suitable heat absorption time, by thermally imaging an unwrapped, planarly arranged sock or fabric with the thermal imaging means (13).

9. A server with a software for diagnosing existence and progression of a disease of patients by imaging and mapping temperatures of human body parts, and analyzing differences between human body parts and changes in view of time, wherein the software is configured to execute the following steps when run in a processor (14, 102):
- capturing at least one thermal image (52) of a human body part from at least one direction by thermal imaging means (13), comprising temperature values in a predetermined area of the human body part, wherein the thermal imaging means is an infrared camera,
- receiving and analyzing thermal image information of the thermal image in a data analysis unit (14, 102), wherein the software is further configured to execute the following step of
- comparing the thermal image information (52) to at least one reference image information by the data analysis unit (14, 102) using artificial intelligence algorithm or algorithms (57), where the reference image information is either a previously taken thermal image of the same human body part of the patient (43), a thermal image of a correspondingly symmetrical part of the human body part of the patient (31, 32, 61), and/or thermal image information picked from a cloud data bank (55, 69, 101) comprising information of all available patients,
**characterized in that** the software is further configured to execute the following steps of:
- merging an optical image (51) of the human body part into the analysis performed by the data analysis unit (14, 102), where the optical image (51) is obtained by a regular camera (12), or by laser scanning,
- performing pattern recognition and pattern classification on the obtained images by the artificial intelligence algorithm or algorithms (57), taking the type or severity of the disease, the patient's age and the patient's weight into analysis to determine a severity of a found thermal deviation in the patient's body, and
- giving a health status parameter by the data analysis unit (14, 102) based on the performed comparison and the merged optical image (51), where the health status parameter is a diabetic progression information or a Charcot based disease status information.

10. The server according to claim 9, **characterized in that** the software is configured to execute the step of:
- cognitively updating the cloud data bank (55, 69, 101) by adding the taken thermal image information (52) together with its deduced parameters, and/or health status determination result given by a doctor into the cloud data bank (55, 69, 101), as part of a learning system in which the server is part of.

## Patentansprüche

1. System zum Diagnostizieren des Vorhandenseins und des Fortschreitens einer Krankheit von Patienten durch Abbilden und Mapping von Temperaturen menschlicher Körperteile und Analysieren von Unterschieden zwischen menschlichen Körperteilen und Änderungen im Hinblick auf die Zeit, wobei das System Folgendes umfasst:
- Wärmebildmittel (13), das konfiguriert ist, mindestens ein Wärmebild (52) eines menschlichen Körperteils aus mindestens einer Richtung aufzunehmen, umfassend Temperaturwerte in einem vorbestimmten Bereich des menschlichen Körperteils, wobei das Wärmebildmittel eine Infrarotkamera ist,
- eine Datenanalyseeinheit (14, 102), die Wärmebildinformationen (52) des Wärmebilds empfängt und analysiert, wobei die Datenanalyseeinheit (14, 102) ferner konfiguriert ist, um
- die Wärmebildinformationen (52) mit mindestens einer Referenzbildinformation unter Verwendung eines Algorithmus oder von Algorithmen künstlicher Intelligenz (57) zu vergleichen, wobei die Referenzbildinformation entweder ein zuvor aufgenommenes Wärmebild desselben menschlichen Körperteils des Patienten (43), ein Wärmebild eines entsprechend symmetrischen Teils des menschlichen Körperteils des Patienten (31, 32, 61) und/oder aus einer Cloud-Datenbank (55, 69, 101) entnommene Wärmebildinformation ist, die Informationen aller verfügbaren Patienten umfasst,
**dadurch gekennzeichnet, dass** die Datenanalyseeinheit (14, 102) ferner konfiguriert ist, um:
- ein optisches Bild (51) des menschlichen Körperteils in eine Analyse einzufügen, die von der Datenanalyseeinheit (14, 102) durchgeführt wird, wobei das optische Bild (51) durch eine gewöhnliche Kamera (12) oder durch Laserabtastung erhalten wird,
- eine Mustererkennung und Musterklassifikation an den erhaltenen Bildern durch den Algorithmus oder die Algorithmen der künstlichen Intelligenz (57) durchzuführen, wobei die Art oder Schwere der Erkrankung, das Alter des Patienten und das Gewicht des Patienten in die Analyse einbezogen werden, um eine Schwere einer gefundenen thermischen Abweichung im Körper des Patienten zu bestimmen; und
- einen Gesundheitszustandsparameter basierend auf dem durchgeführten Vergleich und dem zusammengeführten optischen Bild (51) zu geben, wobei der Gesundheitszustandsparameter eine Information über das Fortschreiten des Diabetes oder eine Information über den Krankheitszustand auf Charcot-Basis ist.

2. System nach Anspruch 1, **dadurch gekennzeichnet, dass** die Datenanalyseeinheit (14, 102) ferner konfiguriert ist, um Cold-Spots, Hot-Spots, thermisch abweichende Bereiche, ihre Temperaturen, Bereichsgrößen und ihre Formen, ihre Nichtsymmetrien und/oder Temperaturgradienten um die thermisch abweichenden Bereiche herum in die Analyse aufzunehmen.

3. System nach Anspruch 1, **dadurch gekennzeichnet, dass** die Datenanalyseeinheit (14, 102) dazu ausgebildet ist, eine abweichende Farbinformation aus dem optischen Bild und/oder mögliche Außenflächendeformationen in die Analyse einzubeziehen.

4. System nach einem der Ansprüche 1 bis 3, **dadurch gekennzeichnet, dass** das System ein lernendes System ist, und das System so konfiguriert ist, dass es die Cloud-Datenbank (55, 69, 101) kognitiv aktualisiert, indem es die aufgenommenen Wärmebildinformationen (52) zusammen mit ihren abgeleiteten Parametern und/oder Ergebnis der Gesundheitszustandsbestimmung durch einen Arzt in die Cloud-Datenbank (55, 69, 101) als Teil des Lernsystems hinzufügt.

5. System nach einem der Ansprüche 1 bis 4, **dadurch gekennzeichnet, dass** das System dazu konfiguriert ist, aufeinanderfolgende Wärmebilder auf zwei gegenüberliegenden Seiten des menschlichen Körperteils aufzunehmen und diese Bilder (66) zu kombinieren, um einen möglichen größeren thermisch abweichenden Bereich in dem menschlichen Körperteil aufzudecken.

6. System nach einem der Ansprüche 1 bis 5, **dadurch gekennzeichnet, dass** das System ein lernendes System ist, und die Wärmebildinformationen (52), die Pixel und ihre Temperaturwerte und abgeleitete Gesundheitszustandsparameter, die durch das System erhalten werden, und möglicherweise manuell aktualisierte Gesundheitszustandsinformationen durch einen untersuchenden Arzt umfassen, so konfiguriert sind, dass sie zu einer Datentabelle als Teil des Lernsystems hinzugefügt werden, die die Wärmebildinformationen (52) in einer Cloud-Datenbank (55, 69, 101) bilden.

7. System nach einem der Ansprüche 1 bis 6, **dadurch gekennzeichnet, dass** das System eine Benutzerschnittstelle (17, 103) umfasst, wo der Benutzer den dynamischen Bereich auswählen kann, der minimale und maximale Temperaturen umfasst, und eine Anzeige (16) so konfiguriert ist, dass sie die Temperaturen des abgebildeten Teils des menschlichen Körpers mit einem Bereich von Farben darstellt, die innerhalb des ausgewählten Dynamikbereichs alternieren.

8. System nach einem der Ansprüche 1 bis 7, **dadurch gekennzeichnet, dass** die Temperaturen des menschlichen Körperteils so konfiguriert sind, dass sie mit einer um den zu messenden Körperteil gelegten oder gewickelten Socke oder Stoff und nach geeigneter Wärmeabsorptionszeit, durch thermisches Abbilden einer unverpackten, flächig angeordneten Socke oder Stoff mit der thermischen Abbildungseinrichtung (13) gemessen werden.

9. Server mit einer Software zur Diagnose des Vorliegens und Fortschreitens einer Erkrankung von Patienten durch Abbildung und Kartierung von Temperaturen menschlicher Körperteile und Analysieren von Unterschieden zwischen menschlichen Körperteilen und Änderungen im Hinblick auf die Zeit, wobei die Software so konfiguriert ist, dass sie die folgenden Schritte ausführt, wenn sie in einem Prozessor (14, 102) läuft:
- Erfassen mindestens eines Wärmebildes (52) eines menschlichen Körperteils aus mindestens einer Richtung durch Wärmebildmittel (13), umfassend Temperaturwerte in einem vorbestimmten Bereich des menschlichen Körperteils, wobei das Wärmebildmittel eine Infrarotkamera ist,
- Empfangen und Analysieren von Wärmebildinformationen des Wärmebilds in einer Datenanalyseeinheit (14, 102), wobei die Software ferner so konfiguriert ist, dass sie den folgenden Schritt ausführt
- Vergleichen der Wärmebildinformationen (52) mit mindestens einer Referenzbildinformation durch die Datenanalyseeinheit (14, 102) unter Verwendung eines Algorithmus oder von Algorithmen (57) künstlicher Intelligenz, wobei die Referenzbildinformation entweder ein zuvor aufgenommenes Wärmebild desselben menschlichen Körperteils des Patienten (43), ein Wärmebild eines entsprechend symmetrischen Teils des menschlichen Körperteils des Patienten (31, 32, 61) und /oder aus einer Cloud-Datenbank (55, 69, 101) entnommene Wärmebildinformation ist, die Informationen aller verfügbaren Patienten umfasst,
**dadurch gekennzeichnet, dass** die Software ferner so konfiguriert ist, dass sie die folgenden Schritte ausführt:
- Einfügen eines optischen Bildes (51) des menschlichen Körperteils in die Analyse, die von der Datenanalyseeinheit (14, 102) durchgeführt wird, wobei das optische Bild (51) durch eine normale Kamera (12) oder durch Laserabtastung erhalten wird,
- Durchführen von Mustererkennung und Musterklassifizierung an den erhaltenen Bildern durch den Algorithmus oder die Algorithmen der künstlichen Intelligenz (57), wobei die Art oder Schwere der Erkrankung, das Alter des Patienten und das Gewicht des Patienten in die Analyse einbezogen werden, um eine Schwere einer gefundenen thermischen Abweichung im Körper des Patienten zu bestimmen, und
- Ausgeben eines Gesundheitszustandsparameters durch die Datenanalyseeinheit (14, 102) basierend auf dem durchgeführten Vergleich und dem zusammengefügten optischen Bild (51), wobei der Gesundheitszustandsparameter eine diabetische Progressionsinformation oder eine auf Charcot basierende Krankheitszustandsinformation ist.

10. Server nach Anspruch 9, **dadurch gekennzeichnet, dass** die Software so konfiguriert ist, dass sie den folgenden Schritt ausführt:
kognitives Aktualisieren der Cloud-Datenbank (55, 69, 101) durch Hinzufügen der aufgenommenen Wärmebildinformationen (52) zusammen mit ihren abgeleiteten Parametern und/oder dem Ergebnis der Gesundheitszustandsbestimmung durch einen Arzt in die Cloud-Datenbank (55, 69, 101), als Teil eines Lernsystems, zu dem der Server gehört.

## Revendications

1. Système pour diagnostiquer l'existence et la progression d'une maladie de patients par imagerie et cartographie des températures de parties du corps humain, et analyse des différences entre les parties du corps humain et des changements de vue dans le temps, dans lequel le système comprend :
- un moyen d'imagerie thermique (13) configuré pour capturer au moins une image thermique (52) d'une partie du corps humain à partir d'au moins une direction, comprenant des valeurs de température dans une zone prédéterminée de la partie du corps humain, dans lequel le moyen d'imagerie thermique est une caméra infrarouge,
- une unité d'analyse de données (14, 102) recevant et analysant des informations d'image thermique (52) de l'image thermique, dans lequel l'unité d'analyse de données (14, 102) est en outre configurée pour comparer les informations d'image thermique (52) à au moins une information d'image de référence à l'aide d'un algorithme ou d'algorithmes d'intelligence artificielle (57), où les informations d'image de référence sont soit une image thermique prise précédemment de la même partie du corps humain du patient (43), une image thermique d'une partie symétrique correspondante de la partie du corps humain du patient (31, 32, 61), et/ou des informations d'image thermique extraites d'une banque de données en nuage (55, 69, 101) comprenant des informations sur tous les patients disponibles,
**caractérisé en ce que** l'unité d'analyse de données (14, 102) est en outre configurée pour :
- fusionner une image optique (51) de la partie du corps humain dans une analyse effectuée par l'unité d'analyse de données (14, 102), où l'image optique (51) est obtenue par une caméra ordinaire (12) ou par balayage laser,
- effectuer une reconnaissance de formes et une classification de formes sur les images obtenues par l'algorithme ou les algorithmes d'intelligence artificielle (57), en prenant le type ou la gravité de la maladie, l'âge du patient et le poids du patient dans l'analyse pour déterminer la gravité d'un écart thermique trouvé dans le corps du patient ; et
- donner un paramètre d'état de santé sur la base de la comparaison effectuée et de l'image optique fusionnée (51), où le paramètre d'état de santé est une information de progression du diabète ou une information d'état de maladie basée sur Charcot.

2. Système selon la revendication 1, **caractérisé en ce que**
l'unité d'analyse de données (14, 102) est en outre configurée pour sélectionner des points froids, des points chauds, des zones thermiquement déviantes, leurs températures, des tailles de zone et leurs formes, leurs non-symétries et/ou leurs gradients thermiques autour des zones thermiquement déviantes dans l'analyse.

3. Système selon la revendication 1, **caractérisé en ce que** l'unité d'analyse de données (14, 102) est configurée pour ajouter une information de couleur déviante de l'image optique et/ou d'éventuelles déformations de surface extérieure dans l'analyse.

4. Système selon l'une quelconque des revendications 1 à 3, **caractérisé en ce que** le système est un système d'apprentissage, et le système est configuré pour mettre à jour de manière cognitive la banque de données en nuage (55, 69, 101) en ajoutant les informations d'image thermique (52) prises conjointement avec ses paramètres déduits, et/ou le résultat de la détermination de l'état de santé donné par un médecin dans la banque de données en nuage (55, 69, 101), dans le cadre du système d'apprentissage.

5. Système selon l'une quelconque des revendications 1 à 4, **caractérisé en ce que** le système est configuré pour prendre des images thermiques ultérieures sur deux côtés opposés de la partie du corps humain, et pour combiner ces images (66) afin de révéler une éventuelle zone de déviation thermique plus grande dans la partie du corps humain.

6. Système selon l'une quelconque des revendications 1 à 5, **caractérisé en ce que** le système est un système d'apprentissage, et les informations d'image thermique (52) comprenant des pixels et leurs valeurs de température, et un paramètre d'état de santé déduit obtenu par le système, et éventuellement des informations d'état de santé mises à jour manuellement par un médecin examinateur, sont configurées pour être ajoutées dans une table de données formant les informations d'image thermique (52) dans une banque de données en nuage (55, 69, 101), dans le cadre du système d'apprentissage.

7. Système selon l'une quelconque des revendications 1 à 6, **caractérisé en ce que** le système comprend une interface utilisateur (17, 103) où l'utilisateur est capable de sélectionner la plage dynamique comprenant des températures minimales et maximales, et un affichage (16) est configuré pour illustrer les températures de la partie imagée du corps humain avec une gamme de couleurs alternant dans la gamme dynamique sélectionnée.

8. Système selon l'une quelconque des revendications 1 à 7, **caractérisé en ce que** les températures de la partie du corps humain sont configurées pour être mesurées avec une chaussette ou un tissu placé ou enroulé autour de la partie du corps à mesurer, et après un temps d'absorption de chaleur approprié, par la formation d'une image thermique d'une chaussette ou d'un tissu disposé dans un plan, non enveloppé, avec le moyen d'imagerie thermique (13) .

9. Serveur avec un logiciel pour diagnostiquer l'existence et la progression d'une maladie de patients par imagerie et cartographie des températures de parties du corps humain, et analyse des différences entre les parties du corps humain et des changements dans le temps, dans lequel le logiciel est configuré pour exécuter les étapes suivantes quand il est exécuté dans un processeur (14, 102) :
- la capture d'au moins une image thermique (52) d'une partie du corps humain à partir d'au moins une direction par des moyens d'imagerie thermique (13), comprenant des valeurs de température dans une zone prédéterminée de la partie du corps humain, dans lequel les moyens d'imagerie thermique sont une caméra infrarouge,
- la réception et l'analyse d'informations d'image thermique de l'image thermique dans une unité d'analyse de données (14, 102), dans lequel le logiciel est en outre configuré pour exécuter l'étape suivante de
- comparaison des informations d'image thermique (52) à au moins une information d'image de référence par l'unité d'analyse de données (14, 102) à l'aide d'un algorithme ou d'algorithmes d'intelligence artificielle (57), où les informations d'image de référence sont soit une image thermique prise précédemment de la même partie du corps humain du patient (43), une image thermique d'une partie symétrique correspondante de la partie du corps humain du patient (31, 32, 61), et/ou des informations d'image thermique extraites d'une banque de données en nuage (55, 69, 101) comprenant des informations sur tous les patients disponibles,
**caractérisé en ce que** le logiciel est en outre configuré pour exécuter les étapes suivantes de :
- fusion d'une image optique (51) de la partie du corps humain dans une analyse effectuée par l'unité d'analyse de données (14, 102), où l'image optique (51) est obtenue par une caméra ordinaire (12) ou par balayage laser,
- réalisation d'une reconnaissance de formes et une classification de formes sur les images obtenues par l'algorithme ou les algorithmes d'intelligence artificielle (57), en prenant le type ou la gravité de la maladie, l'âge du patient et le poids du patient dans l'analyse pour déterminer la gravité d'un écart thermique trouvé dans le corps du patient, et
- fourniture d'un paramètre d'état de santé par l'unité d'analyse de données (14, 102) sur la base de la comparaison effectuée et de l'image optique fusionnée (51), où le paramètre d'état de santé est une information de progression du diabète ou une information d'état de maladie basée sur Charcot.

10. Serveur selon la revendication 9, **caractérisé en ce que** le logiciel est configuré pour exécuter l'étape de :
- mise à jour cognitive de la banque de données en nuage (55, 69, 101) en ajoutant les informations d'image thermique (52) prises conjointement avec ses paramètres déduits, et/ou le résultat de la détermination de l'état de santé donné par un médecin dans la banque de données en nuage (55, 69, 101), dans le cadre d'un système d'apprentissage dont le serveur fait partie.
